# EUROPEAN PATENT APPLICATION

(11) **EP 4 814 561 A1**
(43) Date of publication of application: **30.09.2026**
(21) Application number: 25165568.4
(22) Date of filing: 24.03.2025
(51) Int. Cl.: G02B 21/00, G02B 21/22, G02B 21/36, H04N 13/00

(54) **SYSTEM, METHOD, AND COMPUTER PROGRAM FOR A SURGICAL OR SCIENTIFIC IMAGING SYSTEM AND SURGICAL OR SCIENTIFIC IMAGING SYSTEM**

(71) Applicant: Leica Instruments (Singapore) Pte Ltd., Singapore 618299 (SG)
(72) Inventor: THEMELIS, George, 618299 Singapore (SG)
(74) Representative: 2SPL Patentanwälte PartG mbB

(57) **Abstract**

Examples relate to a system (110), method and computer program for a surgical or scientific imaging system (100), and to a surgical or scientific imaging system comprising such a system. The system is configured to obtain a first image representing a first angle of view on a sample (10) of a first optical imaging sensor (120a), a second image representing a second angle of view on the sample of a second optical imaging sensor (120b), and at least one third image representing at least one third angle of view on the sample of at least one third optical imaging sensor (120c), determine, using the first, second and at least one third image, a three-dimensional depth map of the sample, generate, using at least the first and second image, a view on the sample, provide at least the view on the sample for a display device (130a, 130b) of the surgical or scientific imaging system.

## Description

### Technical field

Examples relate to a system, method, and computer program for a surgical or scientific imaging system, and to a surgical or scientific imaging system comprising the system.

### Background

Traditional surgical microscopes use two imaging channels to create a stereoscopic view, either through eyepieces or cameras. While this stereoscopic imaging can extract three-dimensional surface information via image processing, its accuracy and depth perception are limited by the dual-angle observation. This limitation affects the depth perception and overall quality of 3D mapping of tissues, making it challenging to visualize complex anatomical structures effectively.

Accurate 3D mapping benefits from capturing multiple observation angles, typically achieved through scanning systems that sequentially capture images. Existing 3D scanning systems often require the object being scanned to remain static during image capture, which is impractical in dynamic surgical environments. Additionally, these systems can interfere with the surgical workflow by necessitating changes in observation angles or positioning of the surgical microscope, leading to potential disruptions and distractions during surgery. Moreover, conventional 3D acquisition techniques that capture multiple images sequentially from different angles are time-consuming and require complex setup and calibration. This complexity can increase preparation time, require specialized training, and result in higher costs and maintenance, posing a barrier to widespread adoption. Moreover, many 3D acquisition systems lack the ability to perform real-time 3D reconstruction, resulting in delays in processing and rendering 3D models. This deficiency hinders immediate decision-making and intraoperative guidance, reducing the effectiveness of the surgical procedure.

There may be a desire for an improved concept for three-dimensional mapping of a sample, e.g., for use with a surgical imaging device.

### Summary

This desire is addressed by the subject matter of the independent claims.

The proposed concept is based on the insight that some types of surgical microscopes, such as those used in ophthalmic surgery, utilize more than two imaging channels, allowing simultaneous stereoscopic views for multiple surgeons. Using such imaging device configurations, an arrangement with three or more imaging channels is proposed, with the three or more imaging channels being used to computationally extract a 3D map of the tissue in real-time. The proposed concept leverages multiple imaging observation angles (three, four, or more) to computationally generate a high-quality, detailed, and robust three-dimensional map of the tissue. This process can run in the background in real-time and does not interfere with the primary stereoscopic visualization. The proposed concept may thus provide an improved 3D visualization, providing high-quality, detailed, and robust 3D maps without interrupting the surgical workflow. The proposed concept also allows for real-time processing, as the computational 3D mapping can be performed in the background, ensuring continuous visualization.

Some aspects of the present disclosure relate to a system for a surgical or scientific imaging system. The system comprises one or more processors and one or more storage devices. The system is configured to obtain a first image representing a first angle of view on a sample of a first optical imaging sensor, a second image representing a second angle of view on the sample of a second optical imaging sensor, and at least one third image representing at least one third angle of view on the sample of at least one third optical imaging sensor. The system is configured to determine, using the first, second and at least one third image, a three-dimensional depth map of the sample. The system is configured to generate, using at least the first and second image, a view on the sample. The system is configured to provide at least the view on the sample for a display device of the surgical or scientific imaging system. By using images having three or more angles of view, a robust three-dimensional depth map of the sample can be generated. This three-dimensional depth map can be generated without causing interruptions to the scientific or surgical workflows caused by repositioning of an imaging device of the surgical or scientific imaging system or due to a repositioning of image channels.

In some cases, an angle of view of an imaging channel of one of the optical imaging sensors can be changed, e.g., by laterally moving the imaging channel or by tilting the imaging channel. This can be done automatically or semi-automatically to improve the generation of the three-dimensional depth map, e.g., by selecting an angle of view that allows a better view at an area that is hidden from view for one or two other optical imaging sensors. For example, the system may be configured to, using at least one of the first, second or at least one third image, determine an area of the sample being affected by an obstruction obscuring a view on the sample for at least one of the first, second or at least one third optical imaging sensor. The system may be configured to control the surgical or scientific imaging system to adjust the at least one third angle of view based on the area being affected by the obstruction. This way, the quality and comprehensiveness of the three-dimensional depth map can be improved, e.g., as the obstruction can be avoided with the adjustment of the third angle of view. The proposed concept thus provides versatile imaging options, as additional channels can be dynamically adjusted to improve imaging.

In the proposed concept, the three-dimensional depth map is generated without requiring a repositioning of the imaging device. Even if an angle of view is adjusted, this is done internally, by adjusting an imaging channel being used for the third optical imaging sensor, without requiring an adjustment to the position or orientation of the optics carrier. Accordingly, the system may be configured to control the surgical or scientific imaging system to adjust the at least one third angle of view by changing at least one of a position or an orientation of a channel between the third optical imaging sensor and the sample. This way, the third angle of view can be adjusted without requiring a repositioning of the optics carrier.

In particular, the system may be configured to control the surgical or scientific imaging system to adjust a tilt of the channel(s) between the at least one third optical imaging sensor and the sample based on the area being affected by the obstruction. Additionally, or alternatively, the system may be configured to control the surgical or scientific imaging system to adjust a lateral offset of the channel(s) between the at least one third optical imaging sensor and the sample from channels between the first and second optical imaging sensors and the sample based on the area being affected by the obstruction. These adjustments enable far-reaching adjustments to the at least one third angle of view, e.g., so that the sample can be imaged from an angle of view that avoids an obstruction visible in the first and/or second image.

The use of imaging channels having an adjustable geometry can improve the coverage of the sample, as the respective angle of view can be changed to avoid obstructions. To determine an angle of view that avoids the obstruction, i.e., a desired angle of view, various approaches may be taken. In a first approach, machine learning may be used. In other words, the system may be configured to adjust the at least one third angle of view based on an output of a machine-learning model being trained to output a desired angle of view based on the area of the sample being affected by an obstruction. In general, the determination of a desired angle of view, based on a known current angle of view for the first, second and third optical imaging sensor and based on the known area being obstructed in one (or more) of the images is a finite challenge that can easily be addressed using machine learning.

In a second approach, trigonometry may be used to determine the desired angle of view. In particular, the three-dimensional depth map(s) can be used to determine the position of the area of the sample being obstructed and the position of the object causing the obstruction. Once these positions are known, an angle of view can be selected that avoids the obstruction. In other words, the system may be configured to determine a three-dimensional position of an object causing the obstruction relative to the area of the sample being obstructed, and to adjust the at least one third angle of view based on the three-dimensional position of an object causing the obstruction.

In a third approach, the operator of the surgical or scientific imaging system may be requested to aid in the adjustment of the third angle of view. In other words, the system may be configured to adjust the at least one third angle of view based on an input of a user being prompted to adjust the at least one third angle of view until the obstruction is removed from the third image. This approach may require a slight interruption of the workflow of the operator, and may be useful (e.g., as fallback option) in cases where there is no ideal angle of view for the third image.

The three-dimensional depth map of the sample may be used for various purposes, such as removing or reducing the impact of obstructions that are visible in the first and/or second image. For example, the system may be configured to determine, for at least one of the first image or the second image, an area being affected by an obstruction obscuring a view on the sample. The system may be configured to map one or more image segments of the respective other image or of the third image onto the area being affected by the obstruction using the three-dimensional depth map of the sample. In particular, using the first image, the second image, and optionally the third image, the color and/or intensity at each (three-dimensional) point of the depth map (e.g., each point being obstructed in one of the images) may be determined, and used to fill in the area(s) being obstructed in the view. This way, the view can show the area of the image being obstructed.

To differentiate obstructions from permanent features of the sample, multiple depth maps may be determined and used to identify discrepancies that would indicate an obstruction from one angle of view. For example, the system may be configured to determine the area being affected by an obstruction obscuring a view on the sample based on a depth disparity between at least two of a first depth map being generated starting from the first image, a second depth map being generated starting from the second image or a third depth map being generated starting from the third image. For example, if the depths for a point on the sample differ by at least a threshold value, e.g., at least 1 cm, or at least 10%, it may be assumed that the disparity is caused by an obstruction that obstructs the view on the sample for one of the angles of view (but not the other), which can be compensated for using the proposed concept.

Building surgical or scientific imaging devices with additional imaging channels (having different angles of view) is costly and adds complexity to the respective surgical or scientific imaging device. To avoid such costs or complexities, additional imaging channel(s) may be used that are already used for other purposes, e.g., to provide additional imaging modalities. For example, the system may be configured to obtain the at least one third image of an optical imaging sensor being associated with a different imaging modality than the first and second optical imaging sensors. For example, while the first and second images are obtained from an optical imaging sensor being used for white light imaging, the third optical imaging sensor might be used only for fluorescence imaging.

Accurate 3D mapping benefits from capturing additional observation angles, which can be achieved by sequentially obtaining images from different angles of view. Accordingly, the system may be configured to periodically change the at least one third angle of view to obtain images of the sample from different angles of view. As the adjustments to the third angle of view do not impact the first and second angles of view (which are primarily used for generating the view of the sample), in this way, an improvement in the quality of the three-dimensional depth map can be achieved without impacting the view on the sample.

In some cases, the determination of the three-dimensional depth may be supported by the use of structured light. Generating a three-dimensional map of the sample using structured light includes projecting a series of known patterns, such as stripes or grids, onto the sample. As these patterns illuminate the sample, they are deformed by the surface contours and features of the sample. By capturing images of these deformed patterns from different angles and using computational algorithms to analyze the distortions, the system can reconstruct the three-dimensional map of the sample. Accordingly, the system may be configured to determine the three-dimensional depth map of the sample at least partially based on structured light shown in at least two of the first, second or third image. Furthermore, the system may be configured to control an illumination system to project the structured light pattern onto the surface of the sample. The use of structured light for determining the three-dimensional depth map improves the quality of the three-dimensional mapping procedure, allowing for detailed 3D imaging. The proposed concept provides versatile imaging options, as integrating structured light illumination allows for superior depth perception.

Apart from removing or reducing the impact of obstructions that are visible in the first and/or second image, the three-dimensional depth may also be useful for precisely placing overlays over portions of the sample in the view of the sample. In other words, the system may be configured to generate an overlay based on the three-dimensional depth map of the sample, and to provide the view with the overlay to the stereoscopic display device.

To generate the view of the sample, image information from the images may be combined using image projection. In general, image projection is the process of mapping points from one space into another space. In many cases, image projection is used to project a three-dimensional object onto a two-dimensional image plane, simulating how a two-dimensional camera captures a three-dimensional scene. However, image projection can also be used between two two-dimensional spaces. In the proposed concept, image projection can be used to project pixels between the first and second image, or between the third image and the first or second image, to generate the combined view. For example, keypoint detection algorithms such as SIFT (Scale-Invariant Feature Transform) or SURF (Speeded-Up Robust Features) can be used to identify corresponding features between the respective images. For example, the system may be configured to use the corresponding features between the respective images to determine a mapping between pixels of the respective images. This mapping between the two two-dimensional spaces is called a homography, i.e., a projective transformation between two planes in three-dimensional space. If an area of one of the images is obscured by an obstruction, the system may use the pixels of one of the other images to replace the pixels of the area being obstructed. This way, the view of the sample comprises versions of the first and second image that have been extended (e.g., improved) using pixels of another one of the images. Using a homography for image projection is useful if the sample is largely flat, with the homography accounting for perspective distortion.

More commonly, image projection is used between a three-dimensional representation of an object, such as the sample, and one or more two-dimensional image planes. To generate a view of the sample using image projection, mathematical operations may be used to transform the image information that is contained in the three-dimensional representation of the object (i.e., the sample) into corresponding two-dimensional image information. These mathematical operations are based on the perspective of the respective two-dimensional image relative to the three-dimensional object. This can be done with the help of the depth map, which can be used as a three-dimensional representation of the sample. Thus, the system may be configured to combine the first and second (and optionally the third) images using image projection based on the three-dimensional depth map to generate the view of the sample. In particular, the system may be configured to combine the first, second and third images using image projection based on the three-dimensional depth map to generate the view of the sample. For example, the pixels of the first and second (and optionally the third) images may first be projected onto the three-dimensional depth map, and then reprojected, from the depth map, onto the image planes of the first and second images to generate the view of the sample. This way, obstructions visible in one or both of the first and second images can be compensated for without interrupting the operator's workflow and without altering the microscope's position.

In various examples, the system may be configured to generate a stereoscopic view on the sample and provide the stereoscopic view on the sample to a stereoscopic display device of the surgical or scientific imaging system. This way, the view can be shown via oculars or via a stereoscopic headset of the surgical or scientific imaging system.

Some aspects of the present disclosure relate to a surgical or scientific imaging system. The surgical or scientific imaging system comprises the first optical imaging sensor for generating the first image representing the first angle of view on the sample. The surgical or scientific imaging system comprises the second optical imaging sensor for generating the second image representing the second angle of view on the sample. The surgical or scientific imaging system comprises the at least one third optical imaging sensor for generating the at least one third image representing the at least one third angle of view on the sample. The surgical or scientific imaging system comprises a display device. The surgical or scientific imaging system comprises the above system.

In various examples of the present disclosure, the third angle of view is adjusted, e.g., to enable use of an angle of view that avoids obstructions between the imaging channel and the sample. To enable automatic adjustment of the third angle of view, the adjustment may be performed using a servo motor. Accordingly, the surgical or scientific imaging system may comprise at least one motor configured to adjust the at least one third angle of view on the sample based on a control signal by the system.

In some cases, the determination of the three-dimensional depth may be supported by the use of structured light. Generating a three-dimensional map of sample using structured light includes projecting a series of known patterns, such as stripes or grids, onto the sample. Accordingly, the surgical or scientific imaging system may comprise a structured light emitter configured to emit structured light towards the sample. This way, at least two of the optical imaging sensors may record the projected patterns in the respective images, which are processed by the system.

In some cases, the channel between the third optical imaging sensor and the sample may also be used for projecting the pattern(s) onto the sample. In other words, the structured light emitter may be configured to emit the structured light towards the sample using at least a portion of the channel between the third optical imaging sensor and the sample. This way, a compact construction of the imaging device may be supported.

Some aspects of the present disclosure relate to a corresponding method for a surgical or scientific imaging system. The method comprises obtaining a first image representing a first angle of view on a sample of a first optical imaging sensor, a second image representing a second angle of view on the sample of a second optical imaging sensor, and at least one third image representing at least one third angle of view on the sample of at least one third optical imaging sensor. The method comprises determining, using the first, second and at least one third image, a three-dimensional depth map of the sample. The method comprises generating, using at least the first and second image, a view on the sample. The method comprises providing at least the view on the sample for a display device of the surgical or scientific imaging system.

Another aspect of the present disclosure relates to a computer program having a program code for performing the method when the program is executed on processor.

### Short description of the Figures

Some examples of apparatuses and/or methods will be described in the following by way of example only, and with reference to the accompanying figures, in which:
- Fig. 1a: shows a block diagram of an example of a system for a surgical or scientific imaging system;
- Fig. 1b: shows a schematic diagram of a surgical or scientific imaging system;
- Fig. 1c: shows an example of components an imaging device for a surgical or scientific imaging system with three optical imaging sensors;
- Fig. 2: shows different positions and orientations of a third imaging channel of an imaging device for a surgical or scientific imaging system with three optical imaging sensors;
- Fig. 3: shows two configurations of imaging channels;
- Fig. 4: shows a flow chart of an example of a method for a surgical or scientific imaging system; and
- Fig. 5: shows an example of a system comprising an imaging device and a computer system.

### Detailed Description

Various examples will now be described more fully with reference to the accompanying drawings in which some examples are illustrated.

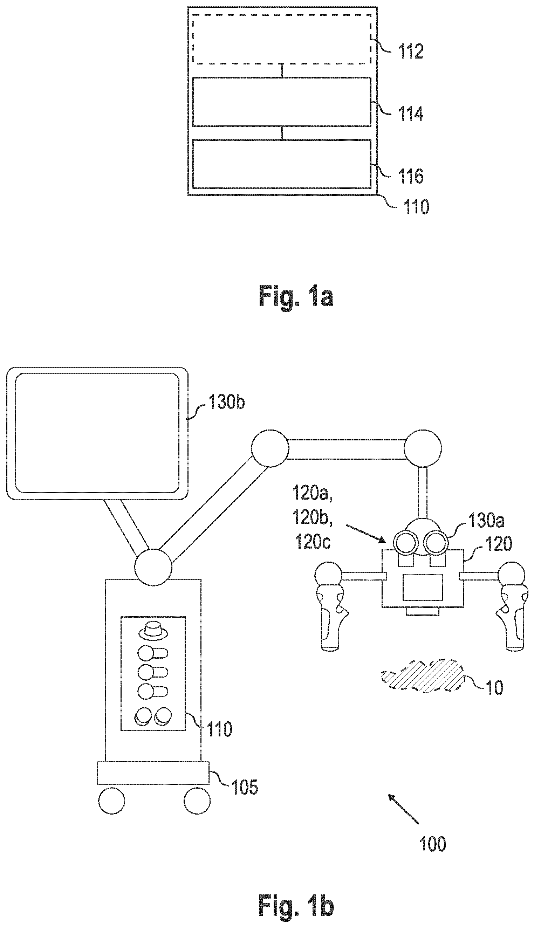

Fig. 1a shows a schematic diagram of an example of a system 110 for a surgical or scientific imaging system 100 (shown in Fig. 1b). The system 110 is a component of the surgical or scientific imaging system 100 and may be used to control various aspects of the surgical or scientific imaging system 100. In particular, it may be used for acquisition and/or processing of images by optical imaging sensors 120a, 120b, 120c of an imaging device 120 of the surgical or scientific imaging system, through various means that will be introduced in more detail in the following. In addition, the system 110 may be configured to control additional aspects of the surgical or scientific imaging system 100, e.g., to provide a display signal for one or more display devices 130a, 130b of the surgical or scientific imaging system.

In general, the system 110 may be a computer system. The system 110 comprises one or more processors 114 and one or more storage devices 116. Optionally, the system 110 further comprises one or more interfaces 112. The one or more processors 114 are coupled to the one or more storage devices 116 and to the one or more interfaces 112. In general, the functionality of the system 110 may be provided by the one or more processors 114, in conjunction with the one or more interfaces 112 (for exchanging data/information with one or more other components of the surgical or scientific imaging system 100 and outside the surgical or scientific imaging system 100, such as an optical imaging sensor of the imaging device 120), and with the one or more storage devices 116 (for storing information, such as machine-readable instructions of a computer program being executed by the one or more processors). In general, the functionality of the one or more processors 114 may be implemented by one or more processors 114 executing machine-readable instructions. Accordingly, any feature ascribed to the one or more processors 114 may be defined by one or more instructions of a plurality of machine-readable instructions. The system 110 may comprise the machine-readable instructions, e.g., within the one or more storage devices 116.

As outlined above, the system 110 is part of the surgical or scientific imaging system 100, which comprises various components in addition to the system 110. For example, the surgical or scientific imaging system 100 comprises the imaging device 120, and may comprise one or more additional components, such as one or more display devices 130a-130b. Fig. 1b shows a schematic diagram of an example of such a surgical or scientific imaging system 100, and in particular of a surgical microscope system 100. In the following, the surgical or scientific imaging system 100 may also be referred to as surgical microscope system 100. A surgical microscope system is a surgical imaging system 100 that comprises a (surgical) microscope as imaging device 120. However, the proposed concept is not limited to such embodiments. The surgical or scientific imaging system 100 may be based on various (single or multiple) imaging devices, such as one or more microscopes, one or more endoscopes, and/or one or more exoscopes (also sometimes called an extracorporeal telescope). Exoscopes are camera-based imaging systems, and in particular camera-based 3D imaging systems, which are suitable for providing images of surgical sites with high magnification and a large depth of field. Compared to microscopes, which may be used via oculars, exoscopes are only used via display modalities, such as a monitor or a head-mounted display. Accordingly, the surgical or scientific imaging system 100 may alternatively be a surgical endoscope system, or a surgical exoscope system. Yet alternatively, the surgical or scientific imaging system may be a scientific imaging system comprising a microscope, e.g., a laboratory microscope. However, the following illustrations assume that the surgical imaging device 120 is a surgical microscope, and that the surgical or scientific imaging system 100 is a surgical microscope system 100.

Accordingly, the surgical or scientific imaging system or surgical microscope system 100 may comprise an imaging device, such as a microscope 120. In general, a microscope, such as the microscope 120, is an optical instrument that is suitable for examining objects that are too small to be examined by the human eye (alone). For example, a microscope may provide an optical magnification of a sample. In the present concept, the optical magnification is (also) provided for at least one optical imaging sensor. The microscope 120 thus comprises an optical imaging sensor, which is coupled with the system 110. The microscope 120 may further comprise one or more optical magnification components that are used to magnify a view on the sample, such as an objective (i.e., lens). For example, the surgical imaging device or microscope 120 is often referred to as the 'optics carrier' of the imaging system.

There are a variety of different types of surgical or scientific imaging devices. If the imaging device is used in the medical or biological fields, the object being viewed through the imaging device may be a sample of organic tissue, e.g., arranged within a petri dish or present in a part of a body of a patient. In various examples presented here, the imaging device 120 may be a microscope of a surgical microscope system, i.e., a microscope that is to be used during a surgical procedure, such as a neurosurgical procedure (i.e., brain surgery). Accordingly, the sample being viewed through the surgical imaging device may be a sample of organic tissue of a patient and may in particular be the surgical site that the surgeon operates on during the surgical procedure, e.g., the brain. However, the proposed concept is also suitable for other types of surgery, such as eye surgery or cardiac surgery.

Fig. 1b shows a schematic diagram of an example of a surgical or scientific imaging system 100, and in particular of a surgical microscope system 100, comprising the system 110 and a microscope 120. The surgical microscope system 100 shown in Fig. 1b comprises a number of optional components, such as a base unit 105 (comprising the system 110) with a (rolling) stand, ocular displays 130a that are arranged at the microscope 120, an auxiliary display 130b that is arranged at the base unit, and the arm that holds the microscope 120 in place, and which is coupled to the base unit and to the microscope 120. In general, these optional and non-optional components may be coupled to the system 110 which may be configured to control and/or interact with the respective components.

In the present case, the system 110 is used to process images of multiple (i.e., three or more) imaging channels to generate a detailed three-dimensional map of the sample. This provides multi-channel generation of 3D surface information, e.g., a multi-view 3D scan microscope for enhanced surgical visualization. The proposed concept thus relates to the utilization of multiple imaging channels in an imaging device, such as a microscope to achieve real-time 3D scanning of samples, such as tissue or surgical cavities. By leveraging multiple observation angles, this system provides superior quality, detail, and robustness in 3D mapping without interrupting the (surgical) workflow.

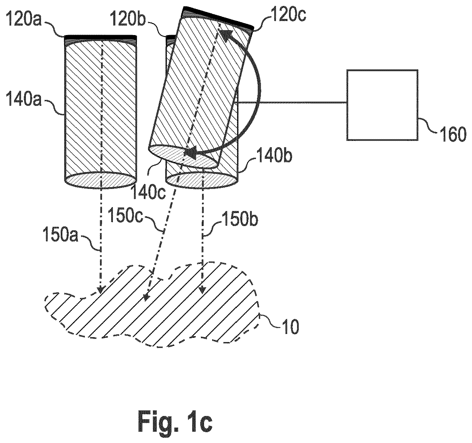

In particular, the system 110 uses images of the sample that show the sample from at least three angles of view. In other words, the system 110 is configured to obtain a first image representing a first angle of view 150a on (e.g., of) a sample 10 of (e.g., from) a first optical imaging sensor 120a, a second image representing a second angle of view 150b on (e.g., of) the sample of (e.g., from) a second optical imaging sensor 120b, and at least one third image representing at least one third angle of view 150c on (e.g., of) the sample of (e.g., from) at least one third optical imaging sensor 120c. Accordingly, the surgical or scientific imaging system 100 (e.g., an imaging device 120 or optics carrier 120 of the surgical or scientific imaging system 100) may comprise the first optical imaging sensor 120a configured to generate the first image, the second optical imaging sensor 120b configured to generate the second image, and the at least one third optical imaging sensor 120c configured to generate the at least one third image. The optical imaging sensors 120a, 120b, 120c, and corresponding imaging channels 140a, 140b, 140c and angles of view 150a, 150b, and 150c are shown in Fig. 1c. Fig. 1c shows an example of components (i.e., the optical imaging sensors 120a, 120b, 120c and corresponding imaging channels 140a, 140b, 140c) of the imaging device 120 of the surgical or scientific imaging system 100, with the imaging device 120 comprising three optical imaging sensors. It is evident from Fig. 1c that the three different optical imaging sensors 120a-c perceive the sample 10, through the imaging channels 140a-c, from different angles of view 150a-c.

The system 110 uses images that show the sample 10 from different angles to generate a depth map of the sample 10. In particular, the system 110 is configured to determine, using the first, second and at least one third image, a three-dimensional depth map of the sample. Moreover, the system 110 provides a digital view on the sample by generating, using at least the first and second images, the (digital) view on the sample, and providing at least the view on the sample for a display device 130a, 130b of the surgical or scientific imaging system 100.

From the perspective of the system 110, the process starts with obtaining the first through third images. The system 110 is configured to obtain (i.e., receive or read out) the images of (e.g., from) the optical imaging sensors 120a-c. The images may be obtained by receiving the images from the optical imaging sensors (e.g., via the interface 112), by reading the images out from a memory of the optical imaging sensors (e.g., via the interface 112), or by reading the images from a storage device 116 of the system 110, e.g., after the images have been written to the storage device 116 by the optical imaging sensors or by another system or processor.

In the proposed concept, at least three images are used that show the sample from at least three angles of view. These images may be provided by different imaging modalities of the imaging device 120. In particular, the first and second optical imaging sensors may be the optical imaging sensors used by a (primary) operator (e.g., surgeon) for stereoscopic viewing of the sample, and the at least one third optical imaging sensor may be an optical imaging sensor that is not (primarily) used by a (primary) operator (e.g., surgeon) for stereoscopic viewing. Thus, if the imaging device 120 of the surgical or scientific imaging system 100 is a stereoscopic imaging device, the system 110 may be configured to obtain stereoscopic imaging data from the first and second optical imaging sensors 120a, 120b of the surgical or scientific imaging system 100, with the stereoscopic imaging data comprising the first image representing a first channel and the second image representing a second channel of the stereoscopic imaging data.

For example, the at least one third optical imaging sensor may comprise at least one optical imaging sensor that is used by a secondary operator (e.g., surgeon). For example, the first and second optical imaging sensors 120a, 120b may be used for stereoscopic viewing by the primary operator, and one or two third optical imaging sensors 120c are used for monoscopic or stereoscopic viewing by the secondary operator being offset from the primary operator by a 90-degree angle. Alternatively, the third optical imaging sensors 120c may be an optical imaging sensor 120c being used for a different imaging modality. In other words, the system 110 may be configured to obtain the at least one third image of an optical imaging sensor 120c being associated with a different imaging modality than the first and second optical imaging sensor. For example, the first and second optical imaging sensors 120a, 120b may be used for white light imaging of the sample, while the third optical imaging sensor 120c is used for generating an overlay based on fluorescence imaging. Alternatively, the third optical imaging sensor may be configured to provide a widefield view of the sample (e.g., of the patient) while the first and second optical imaging sensors 120a, 120b providing the first and second image may be used to provide a magnified view of the sample. Yet alternatively, the at least one third optical imaging sensor 120c may be at least one optical imaging sensor 120 that is arranged to be used (only) for the purpose of generating image(s) to be used for determining the three-dimensional map of the sample. In any case, the additional channel(s) provide complementary observation angle(s), which can enhance the 3D reconstruction process.

The system 110 uses the at least three images to generate the three-dimensional depth map of the sample 10. In particular, the system 110 may be configured to determine the depth map of the sample using stereo disparity analysis based on the first image, second image and third image. Stereo disparity analysis is a technique used to determine the depth information of a scene by measuring the differences in the position of corresponding features between two images taken from slightly different viewing angles (stereo images). To perform stereo disparity analysis with a first image from a first channel and a second image from a second channel, the process typically involves the following tasks. For example, determining the three-dimensional depth map of the sample may comprise identifying and matching corresponding points or features between the two images. This can be done using algorithms like SIFT (Scale-Invariant Feature Transform), SURF (Speeded-Up Robust Features), or other feature detectors and descriptors. Determining the three-dimensional depth map of the sample may further comprise calculating the disparity for each matched pair of points. Disparity is the horizontal difference in position of the corresponding points in the two images. Determining the three-dimensional depth map of the sample may further comprise estimating the three-dimensional depth map of the sample using the calculated disparities, e.g., using triangulation based on the position geometry of the imaging channels.

This process may be repeated for each combination (and reverse combination) of images, e.g., for the following pairs "first image - second image", "first image - third image", "second image - third image", but, in some cases also for "second image - first image", "third image - first image" and "third image - second image", as the result changes depending on which image is used as starting point. For example, the system 110 may be configured to combine (e.g., merge) the depth maps generated using stereo disparity analysis between pairs of images to generate the three-dimensional depth map.

In many cases, in particular in surgical scenarios, there are angles of view from which the view on the sample is obscured by an obstruction, which can impede the generation of the three-dimensional depth map. In particular, the system 110 may be configured to determine, for at least one of the first image or the second image, an area being affected by an obstruction obscuring a view on the sample. In addition, the system may be configured to determine, for the third image, an area being affected by an obstruction obscuring a view on the sample. In general, an area being affected by an obstruction obscuring a view on the sample is an area that is hidden in one or two of the images and visible in the other image(s). This area is specific to each angle of view, as the angle of view determines whether an object is arranged between the respective imaging channel and the sample. Accordingly, the area being affected by an obstruction obscuring a view on the sample may be determined separately for each image (for which the area is determined). For example, blind spots, i.e., the area being affected by an obstruction obscuring a view on the sample, may be detected when two or more pixels from one channel's 2D image (e.g., from the first image, the second image, or the third image) map to the same pixel in another channel's 2D image (e.g., in the respective other image). In other words, the system may be configured to determine the area being affected by an obstruction obscuring a view on the sample by identifying pixels of one of the images that map to the same pixel of another of the images. In this case, depth mapping ensures accurate correspondence between images.

In some examples, the three-dimensional depth map may be used to determine the area being affected by an obstruction. For example, the following approach may be used. In stereo disparity analysis, the resulting three-dimensional depth map changes depending on which of the images is used as a starting point. Swapping the images being used as an input for the stereo disparity analysis can thus lead to different three-dimensional depth maps, which represent the depths as perceived from the respective starting image. In case there is an obstruction that obstructs the view in only one of the images, there is a difference (i.e., a depth disparity) in the three-dimensional depth maps. This difference can be used to detect whether an area is obstructed in one of the images, and also to determine the three-dimensional position of the obstruction (for the image being obstructed by the obstruction). Accordingly, the system 110 may be configured to determine the area being affected by an obstruction obscuring a view on the sample (for one of the images) based on a depth disparity between at least two of a first depth map being generated starting from the first image, a second depth map being generated starting from the second image or a third depth map being generated starting from the third image. This process can be repeated with the three-dimensional depth maps being switched to determine the area being affected by an obstruction obscuring a view on the sample for the respective other image(s).

Determining the area being affected by an obstruction obscuring a view on the sample has multiple applications. A first application relates to the focus of the present application, i.e., the determination of the three-dimensional depth map of the sample. In general, this determination can be improved if the optical imaging sensors have an unobstructed view on the sample. While the first and second optical imaging sensors 120a, 120b are used for stereoscopic viewing of the sample (so that their angles of view might not be changeable without interrupting the task, e.g., surgery, of the primary operator of the surgical or scientific imaging device), in some examples of the present disclosure, changing the third angle of view (without changing the first and second angle of view) is possible.

In particular, the third imaging channel 140c may be adjustable using a motor (motor 160 in Fig. 1c) to improve depth perception and imaging. For example, extra imaging channels (i.e., the at least one third imaging channel 140c), when not used, may be motorized to adjust their position (x, y) and orientation (φ, θ). This adjustability allows for improved complementary angles, resulting in superior 3D acquisition.

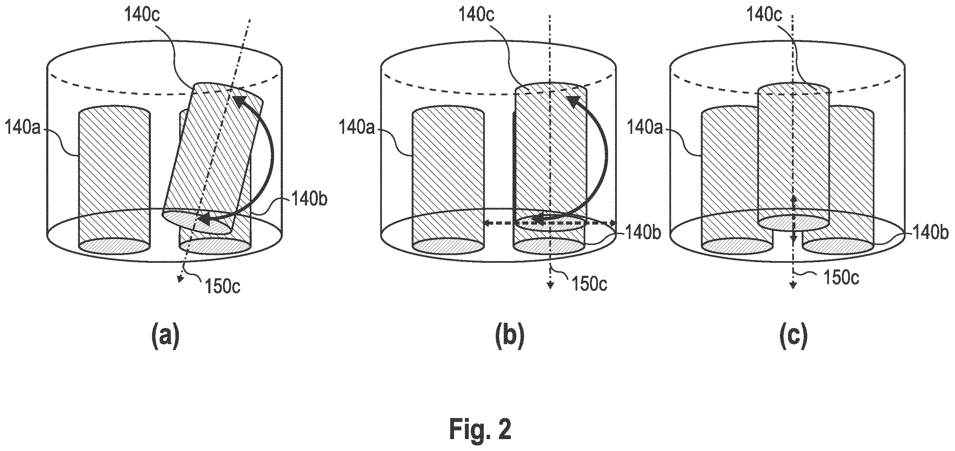

Fig. 2 shows different positions and orientations of a third imaging channel 140c of an imaging device for a surgical or scientific imaging system with three optical imaging sensors. In section (b) of Fig. 2, the third imaging channel is in the default, vertical position, aligned with the primary imaging channels 140a, 140b. In section (a) of Fig. 2, the third imaging channel 140c is in a tilted orientation showing its ability to adjust for optimal complementary angles to the primary channels. In section (c) of Fig. 2, the third imaging channel 140c is repositioned horizontally (i.e., laterally). This adjustment capability enhances the microscope's ability to capture multiple observation angles, contributing to improved 3D reconstruction.

It is evident that this capability can be used to improve the angle of view of the third optical imaging sensor onto the sample, e.g., to allow for improved complementary angles, resulting in an improved generation of the three-dimensional map. This process is controlled by the system 110. Accordingly, the system 110 may be configured to control the surgical or scientific imaging system (e.g., motor 160) to adjust the at least one third angle of view based on the area being affected by the obstruction, e.g., by providing a control signal for the motor 160. Accordingly, the surgical or scientific imaging system may comprise the at least one motor 160 configured to adjust the at least one third angle of view on the sample based on a control signal by the system. In the imaging device 120, servo motor(s) 160 can be used to adjust the lateral position and tilt of an imaging channel by controlling the movement of components, such as the optical lenses or mirrors. By adjusting these components, the servo motor(s) can shift the field of view, and thus the angle of view, laterally or alter the tilt, allowing for fine adjustments in the (third) angle of view 140c without needing to move the entire imaging device. In the present case, as shown in Fig. 2, two kinds of adjustments to the imaging channel 140c are possible - the imaging channel 140c may be moved laterally (see section (c) of Fig. 2), or the orientation of the imaging channel may be adjusted by tilting the imaging channel (see section (a) of Fig. 2). Accordingly, the system 110 may be configured to control the surgical or scientific imaging system (e.g., the motor 160) to adjust the at least one third angle of view by changing at least one of a position or an orientation of the (imaging) channel 140c between the third optical imaging sensor and the sample. In particular, as shown in section (a) of Fig. 2, the system may be configured to control the surgical or scientific imaging system (e.g., the motor 160) to adjust a tilt of the channel 140c between the third optical imaging sensor and the sample based on the area being affected by the obstruction. Additionally, or alternatively, as shown in section (c) of Fig. 2, the system 110 may be configured to control the surgical or scientific imaging system (e.g., the motor 160) to adjust a lateral offset of the channel 140c between the third optical imaging sensor and the sample from channels 140a, 140b between the first and second optical imaging sensors and the sample based on the area being affected by the obstruction.

The aforementioned adjustments to the third angle of view are based on the motivation to improve the angle of view for the third optical imaging sensor 120c. In particular, the adjustment may be performed to avoid or reduce obstruction(s) being visible in the third image, e.g., to improve the determination of the three-dimensional depth map. To determine an angle of view that avoids the obstruction or that improves the determination of the three-dimensional depth map, i.e., the desired angle of view, various approaches may be taken.

In a first approach, machine learning may be used. In other words, the system 110 may be configured to adjust the at least one third angle of view based on an output of a machine-learning model being trained to output a desired angle of view based on the area of the sample being affected by an obstruction. In general, the determination of a desired angle of view, based on a known (current) angle of view for the first, second and third optical imaging sensor, based on the known area being obstructed in one (or more) of the images is a finite challenge that can easily be solved using machine learning. For example, a regression machine learning model may be trained to output the desired angle of view, based on a current third angle of view (being adjusted) and based on the area being affected by an obstruction obscuring a view on the sample. For example, the regression machine learning model may be trained to output, based on the third angle of view (being adjusted) and based on the area being affected by an obstruction obscuring a view on the sample (e.g., based on the area being affected by an obstruction obscuring a view on the sample for the first, second and/or third optical imaging sensor), the desired angle of view for the second or third optical imaging sensor. For example, the regression machine learning model may be trained using supervised learning based on an input comprising the third angle of view (being adjusted) and the area being affected by an obstruction obscuring a view on the sample and a corresponding desired angle of view as a desired result. Alternatively, the regression machine learning model may be trained using reinforcement learning based on a three-dimensional simulation of the area being affected by an obstruction obscuring a view on the sample, with the simulation being parametrized with the angle of view of the third image. A reward function being used for the reinforcement learning may be based on how much the area being affected by the obstruction in the first and/or second image is visible in the third image, for example, or based on a how much of an overall area of the sample is obstructed in the third image, for example.

Alternatively, a three-dimensional simulation may be used to determine the desired angle of view (being used for generating the third image). For example, the system 110 may be configured to determine a three-dimensional position of an object causing the obstruction relative to the area of the sample being obstructed (e.g., based on at least one depth map of the sample). This may be done using stereo disparity analysis, for example. In stereo disparity analysis, the resulting depth map changes depending on which of the images is used as a starting point. Swapping the images being used as an input for the stereo disparity analysis can thus lead to different depth maps, which represent the depths as perceived from the respective starting image. In case there is an obstruction that obstructs the view in only one of the images, there is a difference (i.e., a depth disparity) in the depth maps. This difference can not only be used to detect whether an area is obstructed in one of the images, but also to determine the three-dimensional position of the obstruction (for the image being obstructed by the obstruction). This 3D position of the obstruction may be used to determine the desired angle of view. For example, the system may be configured to adjust the at least one third angle of view based on the three-dimensional position of an object causing the obstruction. For example, the system 110 may be configured to use ray tracing to determine the desired angle of view based on the area of the sample being obstructed and based on the three-dimensional position of the object causing the obstruction.

As a further option, the operator of the surgical or scientific imaging system may be requested to adjust the angle of view (e.g., the second or third angle of view) until visibility of the area being obstructed improves for the imaging channel being adjusted. In other words, the system 110 may be configured to adjust the at least one third angle of view based on an input of a user being prompted to adjust the at least one third angle of view until the obstruction is removed from the third image.

In some cases, a scanning approach may be used, to perform parallel imaging (using the first and second optical imaging sensors 120a, 120b) and scanning (using the third optical imaging sensor 120c). For example, the motorized channels can be used to scan different positions and tilting combinations, acquiring numerous observation images. In other words, the system may be configured to periodically change the at least one third angle of view (e.g., according to a scanning plan comprising a plurality of third angles of views) to obtain images of the sample from different angles of view. These images are combined to produce a more detailed, accurate, and robust 3D model, all while maintaining undisturbed stereoscopic imaging for the primary channels.

Another technique that can be used to improve the precision and reliability of the three-dimensional map of the sample is an integration with structured light illumination, which can be used to improve the depth perception. Accordingly, the system 110 may be configured to determine the three-dimensional depth map of the sample at least partially based on structured light shown in at least two of the first, second or third image, e.g., in addition to, or as an alternative to, the use of stereo disparity analysis. Structured illumination works on the principle that observation from different angles than the illumination source provides additional depth data. Generating a three-dimensional map of the sample using structured light includes projecting a series of known patterns, such as stripes or grids, onto the sample. As these patterns illuminate the sample, they are deformed by the surface contours and features of the sample. By capturing images of these deformed patterns from different angles and using computational algorithms to analyze the distortions, the system can reconstruct the three-dimensional map of the sample. In particular, combining multiple observation angles with structured light illumination enhances the depth information that can be used for generating the three-dimensional depth map. The combination of simultaneous multi-angle acquisition and structured light illumination results in an even more accurate 3D reconstruction.

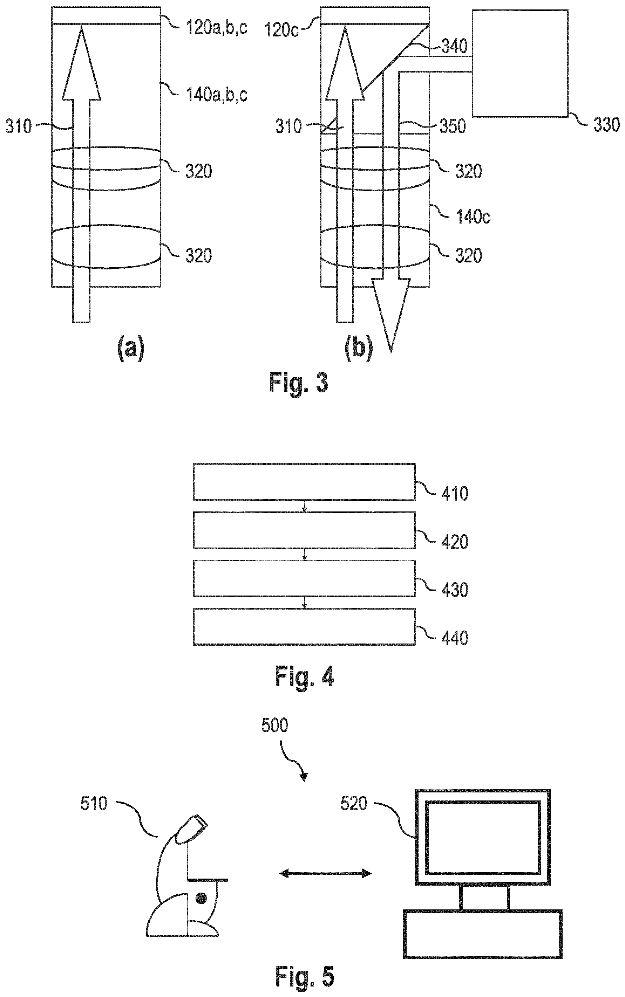

The determination of the depth map with the help of structured light illumination is based on illuminating the sample with light from a structured light source. Accordingly, the surgical or scientific imaging system may comprise a structured light emitter (e.g., structured light emitter 330 shown in section (b) of Fig. 3) configured to emit structured light towards the sample. In general, the structured light emitter may be arranged in or at the imaging device 120, e.g., at a bottom of the imaging device 120, facing the sample, or in the imaging device, with the imaging device 120 comprising an illumination channel between the structured light emitter and the sample. To simplify the imaging system 120, the at least one third channel can double as both an imaging and structured light illumination channel. In other words, the structured light emitter 330 may be configured to emit the structured light towards the sample using at least a portion of a channel between the third optical imaging sensor and the sample. This can be achieved using a beamsplitter to introduce the illumination light into the optical channel, reducing the need for separate illumination hardware.

The use of a beam splitter to introduce the illumination light into the optical channel is shown in Fig. 3. Fig. 3 shows two configurations of imaging channels. Section (a) of Fig. 3 shows an illustration of a standard imaging channel 140a-c used in microscopes, featuring a CMOS sensor 120a-c capturing the imaging light 310. Optical components 320 are arranged in the imaging channel 140a-c. Section (b) of Fig. 3 shows an illustration of a combined imaging and illumination channel 140c. This setup includes a CMOS sensor 120c for imaging and a structured-light generator 330 for illumination. Optical components 320 are arranged in the imaging channel 140c. A beamsplitter 340 integrates the illumination light 350 into the optical path, enabling both imaging and structured-light functions within a single channel.

A major purpose of the proposed concept is to enable (stereoscopic) imaging of the sample and highly-accurate generation of a three-dimensional depth map of the sample at the same time, without generation of the three-dimensional depth map impacting the imaging of the sample. Therefore, the three-dimensional depth map does not necessarily have to be used for generating the view on the sample that is output via a display device 130a, 130b of the surgical or scientific imaging system. Accordingly, the system 110 is configured to generate, using at least the first and second image, a view on the sample, and to provide at least the view on the sample for a display device 130a, 130b of the surgical or scientific imaging system. In particular, the system 110 may be configured to generate a stereoscopic view on the sample and provide the stereoscopic view on the sample to a stereoscopic display device of the surgical or scientific imaging system. In this case, the depth map may be used for other purposes, e.g., for registration of an image guided surgery (IGS) system.

In some cases, the three-dimensional depth map of the sample is used to augment the view on the sample. For example, this can be done by generating an overlay that is based on the three-dimensional depth map. In other words, the system 110 may be configured to generate an overlay based on the three-dimensional depth map of the sample, and to provide the view with the overlay to the display device. For example, the overlay may include information obtained from the aforementioned image guided surgery (IGS) system, or the overlay may include information obtained from an image segmentation or object detection algorithm being used to process the first, second and/or third image. For example, the three-dimensional depth map may be used to accurately place the overlay relative to a corresponding (anatomical) feature of the sample.

The depth map can also be used to combine the images. For example, corresponding image segments from another image (and imaging channel), where an obstruction is not present, may be mapped onto the obstructed areas using the depth map. For example, the system 110 may be configured to combine the first image and the second image (and optionally the third image) using image projection using the three-dimensional depth map to generate the view on the sample. In particular, the system 110 may be configured to combine the first image and the second image (and optionally the third image) using image projection based on the area being affected by an obstruction obscuring a view on the sample, e.g., using the three-dimensional depth map to map a segment of one image onto another image. In other words, the system 110 may be configured to map, using image projection, one or more image segments of the respective other image or of the third image onto the area being affected by the obstruction using the three-dimensional depth map of the sample.

The generation of a composite view that stitches together segments from different images may be used in different contexts (e.g., applications). For example, the technique can be used in a monoscopic application, e.g., to enhance a single 2D image by filling obstructions from one or more other channels (i.e., images). For example, the system 110 may be configured to combine the first image, the second image (and optionally the third image) using image projection using the three-dimensional depth map to obtain a two-dimensional view on the sample, and to provide the view for a 2D monitor 130b of the surgical or scientific imaging system.

Additionally, or alternatively, the proposed concept may be used in a stereoscopic application: It can be applied to both right and left images to create improved stereoscopic pairs for 3D visualization. In other words, the system 110 may be configured to combine the first image, the second image (and optionally the third image) using image projection based on the three-dimensional depth map to obtain a stereoscopic view on the sample, and to provide the stereoscopic view to a stereoscopic display device 130a, such as stereo oculars or a stereo headset, of the surgical or scientific imaging system 100. The same or similar techniques can also be used to supply a 3D monitor of the surgical or scientific imaging system. In other words, the system may be configured to combine the first image and the second image (and optionally the third image) using image projection based on the three-dimensional depth map to obtain a three-dimensional view on the sample, and to provide the view for a 3D monitor 130b of the surgical or scientific imaging system.

In either case, the view is provided as part of a digital view on the sample. This digital view (e.g., of the surgical site) is created and provided to a display device 130a, 130b of the surgical imaging system as part of a display signal. In other words, the system may be configured to output a display signal to the display device 130a, 130b of the surgical or scientific imaging system, with the display signal comprising the view. The view may be viewed by the user, e.g., the surgeon, of the surgical or scientific imaging system. For this purpose, the display signal may be provided to the display device, e.g., an auxiliary display arranged at the base unit of the surgical microscope system, ocular displays integrated within the oculars of the surgical imaging device, or one or two displays of a head-mounted display. For example, the display signal may be a signal for driving (e.g., controlling) the respective display device. For example, the display signal may comprise video data and/or control instructions for driving the display. For example, the display signal may be provided via one of the one or more interfaces 112 of the system. Accordingly, the system 110 may comprise a video interface 112 that is suitable for providing the display signal to the display device 130a, 130b of the microscope system 100.

In the proposed scientific or surgical imaging system, the optical imaging sensors 120a, 120b, 120c are used to provide the aforementioned images. Accordingly, the optical imaging sensors, which may be part of the imaging device 120 (e.g., of the microscope) may be configured to generate the images. For example, the optical imaging sensors of the imaging device 120 may comprise or be an APS (Active Pixel Sensor) - or a CCD (Charge-Coupled-Device)-based imaging sensor. For example, in APS-based imaging sensors, light is recorded at each pixel using a photodetector and an active amplifier of the pixel. APS-based imaging sensors are often based on CMOS (Complementary Metal-Oxide-Semiconductor) or S-CMOS (Scientific CMOS) technology. In CCD-based imaging sensors, incoming photons are converted into electron charges at a semiconductor-oxide interface, which are subsequently moved between capacitive bins in the imaging sensors by a circuitry of the imaging sensors to perform the imaging.

The one or more interfaces 112 of the system 110 may correspond to one or more inputs and/or outputs for receiving and/or transmitting information, which may be in digital (bit) values according to a specified code, within a module, between modules or between modules of different entities. For example, the one or more interfaces 112 may comprise interface circuitry configured to receive and/or transmit information. The one or more processors 114 of the system 110 may be implemented using one or more processing units, one or more processing devices, any means for processing, such as a processor, a computer or a programmable hardware component being operable with accordingly adapted software. In other words, the described function of the one or more processors 114 may as well be implemented in software, which is then executed on one or more programmable hardware components. Such hardware components may comprise a general-purpose processor, a Digital Signal Processor (DSP), a micro-controller, etc. The one or more storage devices 116 of the system 110 may comprise at least one element of the group of a computer readable storage medium, such as a magnetic or optical storage medium, e.g., a hard disk drive, a flash memory, Floppy-Disk, Random Access Memory (RAM), Programmable Read Only Memory (PROM), Erasable Programmable Read Only Memory (EPROM), an Electronically Erasable Programmable Read Only Memory (EEPROM), or a network storage.

More details and aspects of the system 110 and of the surgical or scientific imaging system 100 are mentioned in connection with the proposed concept or one or more examples described above or below (e.g. Fig. 4 to 5). The system 110 and the surgical or scientific imaging system 100 may comprise one or more additional optional features corresponding to one or more aspects of the proposed concept or one or more examples described above or below.

Fig. 4 shows a flow chart of an example of a corresponding method for a surgical or scientific imaging system. For example, the method may be performed by the surgical or scientific imaging system 100 shown in Fig. 1b, and in particular by the system 110 shown in Figs. 1a and 1b. The method comprises obtaining 410 a first image representing a first angle of view on a sample of a first optical imaging sensor, a second image representing a second angle of view on the sample of a second optical imaging sensor, and at least one third image representing at least one third angle of view on the sample of at least one third optical imaging sensor. The method comprises determining 420, using the first, second and at least one third image, a three-dimensional depth map of the sample. The method comprises generating 430, using at least the first and second image, a view on the sample. The method comprises providing 440 at least the view on the sample for a display device of the surgical or scientific imaging system. Features discussed in connection with the surgical or scientific imaging system of Figs. 1a to 1c and 2 to 3 may likewise be included in the method of Fig. 4.

More details and aspects of the method are mentioned in connection with the proposed concept, or one or more examples described above or below (e.g., Fig. 1a to 3, 5). The method may comprise one or more additional optional features corresponding to one or more aspects of the proposed concept, or one or more examples described above or below.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

Some embodiments relate to an imaging device or imaging system, such as the surgical or scientific imaging device 120 or the surgical or scientific imaging system 100 of Fig. 1a and/or 1b comprising a system 110 as described in connection with one or more of the Figs. 1a to 4. Alternatively, an imaging device, such as a microscope or an exoscope, may be part of or connected to a system as described in connection with one or more of the Figs. 1a to 4. Fig. 5 shows a schematic illustration of a system 500 configured to perform a method described herein. The system 500 comprises an imaging device 510, such as a microscope or exoscope, and a computer system 520. The imaging device 510 is configured to take images and is connected to the computer system 520. The computer system 520 is configured to execute at least a part of a method described herein. The computer system 520 may be configured to execute a machine learning algorithm. The computer system 520 and microscope 510 may be separate entities but can also be integrated together in one common housing. The computer system 520 may be part of a central processing system of the imaging device 510 and/or the computer system 520 may be part of a subcomponent of the imaging device 510, such as a sensor, an actor, a camera or an illumination unit, etc. of the imaging device 510.

The computer system 520 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 520 may comprise any circuit or combination of circuits. In one embodiment, the computer system 520 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit. Other types of circuits that may be included in the computer system 520 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 520 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 520 may also include a display device, one or more speakers, and a keyboard and/or controller, which can include a mouse, trackball, touch screen, voice-recognition device, or any other device that permits a system user to input information into and receive information from the computer system 520.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. **The** implementation can be performed using a nontransitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. **The** program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all the functionalities of the methods described herein. In some embodiments, a field programmable gate array may cooperate with a microprocessor to perform one of the methods described herein. Generally, the methods are preferably performed by any hardware apparatus.

Embodiments may be based on using a machine-learning model or machine-learning algorithm. Machine learning may refer to algorithms and statistical models that computer systems may use to perform a specific task without using explicit instructions, instead relying on models and inference. For example, in machine-learning, instead of a rule-based transformation of data, a transformation of data may be used, that is inferred from an analysis of historical and/or training data. For example, the content of images may be analyzed using a machine-learning model or using a machine-learning algorithm. In order for the machine-learning model to analyze the content of an image, the machine-learning model may be trained using training images as input and training content information as output. By training the machine-learning model with a large number of training images and/or training sequences (e.g. words or sentences) and associated training content information (e.g. labels or annotations), the machine-learning model "learns" to recognize the content of the images, so the content of images that are not included in the training data can be recognized using the machine-learning model. The same principle may be used for other kinds of sensor data as well: By training a machine-learning model using training sensor data and a desired output, the machine-learning model "learns" a transformation between the sensor data and the output, which can be used to provide an output based on non-training sensor data provided to the machine-learning model. The provided data (e.g. sensor data, meta data and/or image data) may be preprocessed to obtain a feature vector, which is used as input to the machine-learning model.

Machine-learning models may be trained using training input data. The examples specified above use a training method called "supervised learning". In supervised learning, the machine-learning model is trained using a plurality of training samples, wherein each sample may comprise a plurality of input data values, and a plurality of desired output values, i.e. each training sample is associated with a desired output value. By specifying both training samples and desired output values, the machine-learning model "learns" which output value to provide based on an input sample that is similar to the samples provided during the training. Apart from supervised learning, semi-supervised learning may be used. In semi-supervised learning, some of the training samples lack a corresponding desired output value. Supervised learning may be based on a supervised learning algorithm (e.g. a classification algorithm, a regression algorithm or a similarity learning algorithm. Classification algorithms may be used when the outputs are restricted to a limited set of values (categorical variables), i.e. the input is classified to one of the limited set of values. Regression algorithms may be used when the outputs may have any numerical value (within a range). Similarity learning algorithms may be similar to both classification and regression algorithms but are based on learning from examples using a similarity function that measures how similar or related two objects are. Apart from supervised or semi-supervised learning, unsupervised learning may be used to train the machine-learning model. In unsupervised learning, (only) input data might be supplied and an unsupervised learning algorithm may be used to find structure in the input data (e.g. by grouping or clustering the input data, finding commonalities in the data). Clustering is the assignment of input data comprising a plurality of input values into subsets (clusters) so that input values within the same cluster are similar according to one or more (pre-defined) similarity criteria, while being dissimilar to input values that are included in other clusters.

Reinforcement learning is a third group of machine-learning algorithms. In other words, reinforcement learning may be used to train the machine-learning model. In reinforcement learning, one or more software actors (called "software agents") are trained to take actions in an environment. Based on the taken actions, a reward is calculated. Reinforcement learning is based on training the one or more software agents to choose the actions such, that the cumulative reward is increased, leading to software agents that become better at the task they are given (as evidenced by increasing rewards).

Furthermore, some techniques may be applied to some of the machine-learning algorithms. For example, feature learning may be used. In other words, the machine-learning model may at least partially be trained using feature learning, and/or the machine-learning algorithm may comprise a feature learning component. Feature learning algorithms, which may be called representation learning algorithms, may preserve the information in their input but also transform it in a way that makes it useful, often as a pre-processing step before performing classification or predictions. Feature learning may be based on principal components analysis or cluster analysis, for example.

In some examples, anomaly detection (i.e. outlier detection) may be used, which is aimed at providing an identification of input values that raise suspicions by differing significantly from the majority of input or training data. In other words, the machine-learning model may at least partially be trained using anomaly detection, and/or the machine-learning algorithm may comprise an anomaly detection component.

In some examples, the machine-learning algorithm may use a decision tree as a predictive model. In other words, the machine-learning model may be based on a decision tree. In a decision tree, observations about an item (e.g. a set of input values) may be represented by the branches of the decision tree, and an output value corresponding to the item may be represented by the leaves of the decision tree. Decision trees may support both discrete values and continuous values as output values. If discrete values are used, the decision tree may be denoted a classification tree, if continuous values are used, the decision tree may be denoted a regression tree.

Association rules are a further technique that may be used in machine-learning algorithms. In other words, the machine-learning model may be based on one or more association rules. Association rules are created by identifying relationships between variables in large amounts of data. The machine-learning algorithm may identify and/or utilize one or more relational rules that represent the knowledge that is derived from the data. The rules may e.g. be used to store, manipulate or apply the knowledge.

Machine-learning algorithms are usually based on a machine-learning model. In other words, the term "machine-learning algorithm" may denote a set of instructions that may be used to create, train or use a machine-learning model. The term "machine-learning model" may denote a data structure and/or set of rules that represents the learned knowledge (e.g. based on the training performed by the machine-learning algorithm). In embodiments, the usage of a machine-learning algorithm may imply the usage of an underlying machine-learning model (or of a plurality of underlying machine-learning models). The usage of a machine-learning model may imply that the machine-learning model and/or the data structure/set of rules that is the machine-learning model is trained by a machine-learning algorithm.

For example, the machine-learning model may be an artificial neural network (ANN). ANNs are systems that are inspired by biological neural networks, such as can be found in a retina or a brain. ANNs comprise a plurality of interconnected nodes and a plurality of connections, so-called edges, between the nodes. There are usually three types of nodes, input nodes that receiving input values, hidden nodes that are (only) connected to other nodes, and output nodes that provide output values. Each node may represent an artificial neuron. Each edge may transmit information, from one node to another. The output of a node may be defined as a (non-linear) function of its inputs (e.g. of the sum of its inputs). The inputs of a node may be used in the function based on a "weight" of the edge or of the node that provides the input. The weight of nodes and/or of edges may be adjusted in the learning process. In other words, the training of an artificial neural network may comprise adjusting the weights of the nodes and/or edges of the artificial neural network, i.e. to achieve a desired output for a given input.

Alternatively, the machine-learning model may be a support vector machine, a random forest model or a gradient boosting model. Support vector machines (i.e. support vector networks) are supervised learning models with associated learning algorithms that may be used to analyze data (e.g. in classification or regression analysis). Support vector machines may be trained by providing an input with a plurality of training input values that belong to one of two categories. The support vector machine may be trained to assign a new input value to one of the two categories. Alternatively, the machine-learning model may be a Bayesian network, which is a probabilistic directed acyclic graphical model. A Bayesian network may represent a set of random variables and their conditional dependencies using a directed acyclic graph. Alternatively, the machine-learning model may be based on a genetic algorithm, which is a search algorithm and heuristic technique that mimics the process of natural selection.

### List of Reference Numerals

- 10:: Sample
- 100:: Surgical or scientific imaging system
- 105:: Base unit
- 110:: System
- 112:: One or more interfaces
- 114:: One or more processors
- 116:: One or more storage devices
- 120:: Imaging device
- 120a, 120b, 120c:: Optical imaging sensors
- 130a:: Ocular displays
- 130b:: Auxiliary display
- 140a, 140b, 140c:: Imaging channels
- 150a, 150b, 150c:: Angles of view
- 160:: Motor
- 310:: Imaging light
- 320:: Optical component
- 330:: Structured-light generator
- 340:: Beamsplitter
- 350:: Illumination light (structured light)
- 410:: Obtaining a first, second, and third image
- 420:: Determining a three-dimensional depth map
- 430:: Generating a view on a sample
- 440:: Providing at least the view for a display device
- 500:: System
- 510:: Imaging device
- 520:: Computer system

## Claims

1. A system (110) for a surgical or scientific imaging system (100), the system comprising one or more processors and one or more storage devices, wherein the system is configured to:
obtain a first image representing a first angle of view (150a) on a sample (10) of a first optical imaging sensor (120a), a second image representing a second angle of view (150b) on the sample of a second optical imaging sensor (120b), and at least one third image representing at least one third angle of view (150c) on the sample of at least one third optical imaging sensor (120c),
determine, using the first, second and at least one third image, a three-dimensional depth map of the sample;
generate, using at least the first and second image, a view on the sample;
provide at least the view on the sample for a display device (130a, 130b) of the surgical or scientific imaging system.

2. The system according to claim 1, wherein the system is configured to, using at least one of the first, second or at least one third image, determine an area of the sample being affected by an obstruction obscuring a view on the sample for at least one of the first, second or at least one third optical imaging sensor, and to control the surgical or scientific imaging system to adjust the at least one third angle of view based on the area being affected by the obstruction.

3. The system according to claim 2, wherein the system is configured to control the surgical or scientific imaging system to adjust the at least one third angle of view by changing at least one of a position or an orientation of a channel (140c) between the third optical imaging sensor and the sample.

4. The system according to one of the claims 2 or 3, wherein the system is configured to control the surgical or scientific imaging system to adjust at least one of a tilt of the channel or channels (140c) between the at least one third optical imaging sensor and the sample or a lateral offset of the channel or channels (140c) between the third optical imaging sensor and the sample from channels (140a, 140b) between the first and second optical imaging sensors and the sample based on the area being affected by the obstruction.

5. The system according to one of the claims 2 to 4, wherein the system is configured to adjust the at least one third angle of view based on an output of a machine-learning model being trained to output a desired angle of view based on the area of the sample being affected by an obstruction,
or wherein the system is configured to determine a three-dimensional position of an object causing the obstruction relative to the area of the sample being obstructed, and to adjust the at least one third angle of view based on the three-dimensional position of an object causing the obstruction,
or wherein the system is configured to adjust the at least one third angle of view based on an input of a user being prompted to adjust the at least one third angle of view until the obstruction is removed from the third image.

6. The system according to one of the claims 1 to 5, wherein the system is configured to determine, for at least one of the first image or the second image, an area being affected by an obstruction obscuring a view on the sample, and to map one or more image segments of the respective other image or of the third image onto the area being affected by the obstruction using the three-dimensional depth map of the sample.

7. The system according to one of the claims 2 to 6, wherein the system is configured to determine the area being affected by an obstruction obscuring a view on the sample based on a depth disparity between at least two of a first depth map being generated starting from the first image, a second depth map being generated starting from the second image or a third depth map being generated starting from the third image.

8. The system according to one of the claims 1 to 7, wherein the system is configured to obtain the at least one third image of an optical imaging sensor being associated with a different imaging modality than the first and second optical imaging sensor.

9. The system according to one of the claims 1 to 8, wherein the system is configured to periodically change the at least one third angle of view to obtain images of the sample from different angles of view.

10. The system according to one of the claims 1 to 9, wherein the system is configured to determine the three-dimensional depth map of the sample at least partially based on structured light shown in at least two of the first, second or third image.

11. **The** system according to one of the claims 1 to 10, wherein the system is configured to generate an overlay based on the three-dimensional depth map of the sample, and to provide the view with the overlay to the display device,
or wherein the system is configured to combine the first and second image or the first, second and third image using image projection based on the three-dimensional depth map to generate the view of the sample.

12. **The** system according to one of the claims 1 to 11, wherein the system is configured to generate a stereoscopic view on the sample and provide the stereoscopic view on the sample to a stereoscopic display device of the surgical or scientific imaging system.

13. A surgical or scientific imaging system (100), comprising
a first optical imaging sensor (120a) for generating a first image representing a first angle of view (150a) on a sample (10);
a second optical imaging sensor (120b) for generating a second image representing a second angle of view (150b) on the sample;
at least one third optical imaging sensor (120c) for generating at least one third image representing at least one third angle of view on the sample (150c);
a display device (130a, 130b); and
the system (110) according to one of the claims 1 to 12.

14. A method for a surgical or scientific imaging system (100), the method comprising:
obtaining (410) a first image representing a first angle of view (150a) on a sample (10) of a first optical imaging sensor (120a), a second image representing a second angle of view (150b) on the sample of a second optical imaging sensor (120b), and
at least one third image representing at least one third angle of view (150c) on the sample of at least one third optical imaging sensor (120c),
determining (420), using the first, second and at least one third image, a three-dimensional depth map of the sample;
generating (430), using at least the first and second image, a view on the sample; and
providing (440) at least the view on the sample for a display device (130a, 130b) of the surgical or scientific imaging system.

15. A computer program having a program code for performing a method according to claim 14 when the program is executed on processor.
